# EUROPEAN PATENT SPECIFICATION

(11) **EP 1 548 425 B1**
(45) Date of publication and mention of the grant of the patent: **18.05.2011**
(21) Application number: 03794289.3
(22) Date of filing: 08.09.2003
(51) Int. Cl.: G01N 21/78, G01N 21/01, G01N 31/22, G01N 33/52, G01N 33/50

(54) **Test kit**
Testkit
Kit de test

(30) Priority: 09.09.2002 JP 2002262510
(43) Date of publication of application: 29.06.2005
(73) Proprietor: ARKRAY, Inc., Kyoto-shi, Kyoto 601-8045 (JP)
(72) Inventor: MATSUDA, Takeshi, ARKRAY, Inc., Kyoto-shi, Kyoto 601-8045 (JP); OKUBO, Akio, ARKRAY, Inc., Kyoto-shi, Kyoto 601-8045 (JP)
(74) Representative: Jones, Elizabeth Louise
(86) International application number: PCT/JP2003/011462
(87) International publication number: WO 2004/023121

(56) References cited:
- EP-A- 0 407 800
- EP-A- 1 118 859
- WO-A-02/063296
- WO-A1-00/42430
- JP-A- 6 213 886
- JP-A- 2000 146 959
- JP-A- 2001 330 606
- JP-A- 2002 071 684
- US-A- 4 839 296
- US-B1- 6 258 045
- ANONYMOUS: "Cyclopore Membranes"[Online] 19 January 1998 (1998-01-19), XP002397263 Retrieved from the Internet: URL:http://web.archive.org/web/19980119135 735/whatman.com/product/lf4e.htm> [retrieved on 2006-09-01]
- ANONYMOUS: "Anodisc Membranes"[Online] 19 January 1998 (1998-01-19), XP002397264 Retrieved from the Internet: URL:http://web.archive.org/web/19980119135 507/whatman.com/product/lf4a.htm> [retrieved on 2006-09-01]
- PATENT ABSTRACTS OF JAPAN vol. 2000, no. 08, 6 October 2000 (2000-10-06) -& JP 2000 146959 A (KDK CORP), 26 May 2000 (2000-05-26)

## Description

This invention relates to a test kit used in the analysis of specific components in a sample liquid.

### BACKGROUND ART

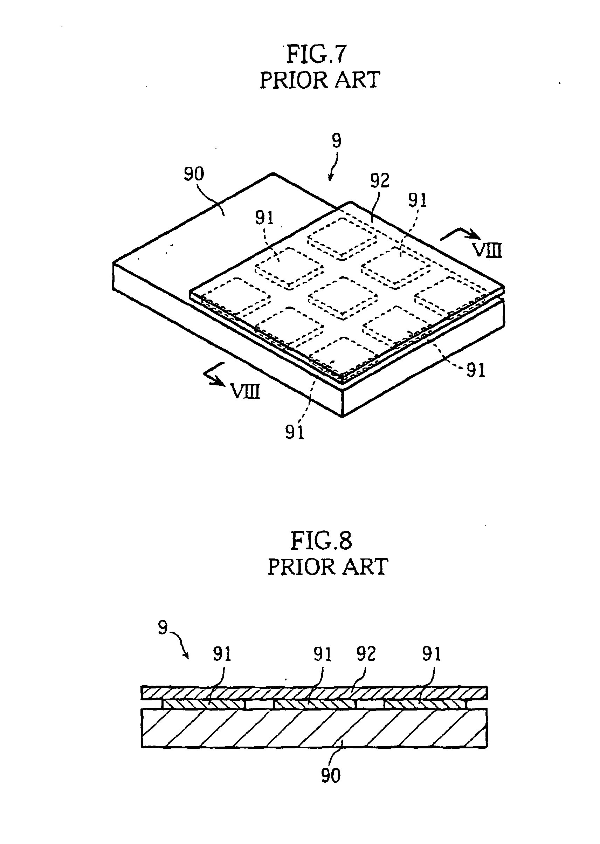

With a dry test kit, as shown in Figs. 7 and 8, a plurality of coloration pads 91 are arranged in a matrix on a carrier 90. All of the coloration pads 91 are covered by a single spreading penetration layer 92. With this dry test kit 9, when the spreading penetration layer 92 is spotted with a sample liquid, the sample liquid spreads out in the planar direction of the spreading penetration layer 92 while penetrating into the spreading penetration layer 92 in the thickness direction. As a result, all of the sample liquid is supplied to the coloration pads 91.

with the dry test kit 9, because the spreading penetration layer 92 allows the sample liquid to spread out in the planar direction after the supply of the sample liquid, liquid junctions may be formed between adjacent coloration pads 91, resulting in interference between these coloration pads 91. In other words, the coloring substance that seeps out of one coloration pad 91 may be admixed into an adjacent coloration pad 91 via the spreading penetration layer 92.

One possible way to suppress such interference is to ensure plenty of distance between adjacent coloration pads 91. Nevertheless, if the distance between the coloration pads 91 is increased, this makes the dry test kit 9 larger, and more sample liquid is required for testing.

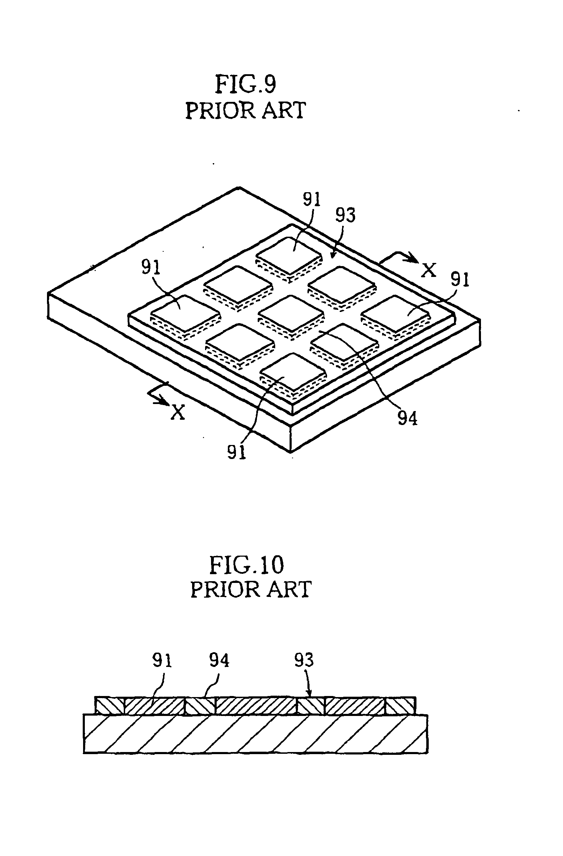

Another possible way to suppress interference, as shown in Figs. 9 and 10, is to provide a partition 93 between adjacent coloration pads 91 (see, for example, Japanese Laid-Open Patent Application 2002-71684), or to provide a water repellant layer between adjacent coloration pads 91 (see, for example, Japanese Laid-Open Patent Application 2001-349835).

However, providing a partition 93 or a water repellency treatment is disadvantageous in terms of both cost and work efficiency in the production of the test kit. Also, the coloration pads 91 need to be formed small in order to keep the test kit to a compact size, but this requires that the width of the partition 93 also be smaller. Accordingly, when the coloration pads 91 are formed in a small size of 1 mm square, for example, a costly apparatus has to be used to form the partition 93, which is disadvantageous in terms of producing cost.

It is known from WO 02/063296 to provide a test kit comprising a distribution layer to which a sample is applied, a filtering layer in the form of a glass fiber membrane, and a plurality of reagent pads in contact with the filtering layer.

It is known from WO 00/42430 to provide a test kit having a first layer comprising a wicking member and a second layer comprising a capillary transfer plane member. The capillary transfer plane has a plurality of capillary transfer passages. The capillary transfer passages can comprise a hole, channel, or other shape to control sample flow rate. Located above one or more of the capillary transfer passages are a plurality of testing members.

### DISCLOSURE OF THE INVENTION

It is an object of the present invention to allow an analytical kit comprising a plurality of coloration pads to be produced at low cost, and to suppress interference between adjacent coloration pads while also keeping the size of the test kit small

The present invention achieves this by providing a test kit comprising: a water absorbent carrier for receiving a sample liquid and for spreading it out, a penetration layer for drawing up the sample liquid, and a plurality of coloration pads held in contact with the penetration layer, wherein the penetration layer and the plurality of coloration pads are laminated in this order on the water absorbent carrier; wherein the penetration layer is arranged for feeding the sample liquid to each of the coloration pads; wherein the penetration layer is formed with a plurality of thicknesswise extending pores for allowing the sample liquid to penetrate thicknesswise of said penetration layer while restricting the sample liquid from spreading in a planar direction of the penetration layer;

characterised in that the plurality of pores have a size of 0.1-12 µm, in that the penetration layer is a track etched membrane, in that the plurality of coloration pads are formed within a specific region, the surface area of the specific region being 2.0~15mm x 2.0~15mm, and in that the surface area of the specific region accounted for by each respective coloration pad is no more than 2.0 mm².

The penetration layer and the plurality of coloration pads are laminated in this order on a water absorbent carrier. The sample liquid supplied to the water absorbent carrier is supplied to the coloration pads through the penetration layer. The water absorbent carrier can be a porous material, for example. Examples of porous materials that can be used include paper materials, foams, woven fabric materials, nonwoven fabric materials, knit materials, glass filters, and gelled materials.

The porosity of the penetration membrane is set to lie in the range of 4-20vol% for example.

The penetration membrane is a track etched membrane formed by track etching. Track etching is a method in which a polymer film is bombarded with neutrons, and pores are formed by chemical etching. With this method, the pore size and porosity can be controlled by varying the neutron bombardment time or the etching treatment time.

The plurality of coloration pads are arranged in a matrix, for example, but the plurality of coloration pads can also be arranged in a straight line.

The analytical kit of the present invention is typically designed such that at least two of the plurality of coloration pads differ from each other with respect to coloration components for allowing measurement of a plurality of items.

Also described is an example of a method for producing a test kit, which comprises a first step of forming a plurality of coloration pads by coating a carrier with a reagent liquid containing a coloration component using a non-contact dispenser and by thereafter drying the reagent liquid, and a second step of intimately attaching a penetration membrane so as to cover the plurality of coloration pads. The penetration membrane used in the second step allows liquid penetration thicknesswise of the penetration membrane while restricting liquid penetration in the planar direction of the penetration membrane.

The penetration membrane can be the same as that described above.

The non-contact dispenser used in the first step is of an inkjet type. The non-contact dispenser may also be one that employs a dispenser discharge system.

In the first step, the plurality of coloration pads are formed in a matrix arrangement for example. The plurality of coloration pads may instead be formed so as to be linearly aligned.

In the first step, at least two of the plurality of coloration pads may differ from each other with respect to coloration components. Specifically, the present invention is applicable to the production of an analytical kit capable of analyzing a plurality of items.

In the first step, the plurality of coloration pads are formed within a specific region with a surface area of 2.0~15mm×2.0~15mm. The surface area of the specific region accounted for by the respective coloration pads is no more than 2.0mm².

### BRIEF DESCRIPTION OF THE DRAWINGS

Fig. 1 is an overall oblique view of a test kit which is not an embodiment of the present invention;
Fig. 2 is a cross section along the II-II line in Fig. 1;
Fig. 3 is a cross section illustrating an analysis method that makes use of the test kit shown in Figs. 1 and 2;
Fig. 4 is a cross section illustrating an analysis method that makes use of the test kit shown in Figs. 1 and 2;
Fig. 5 is an overall oblique view of a test kit pertaining to an embodiment of the present invention;
Fig. 6 is a cross section illustrating the action of the test kit shown in Fig. 5;
Fig. 7 is an overall oblique view of a conventional test kit;
Fig. 8 is a cross section along the VIII-VIII line in Fig. 7;
Fig. 9 is an overall oblique view of another example of a conventional test kit; and
Fig. 10 is a cross section along the X-X line in Fig. 9.

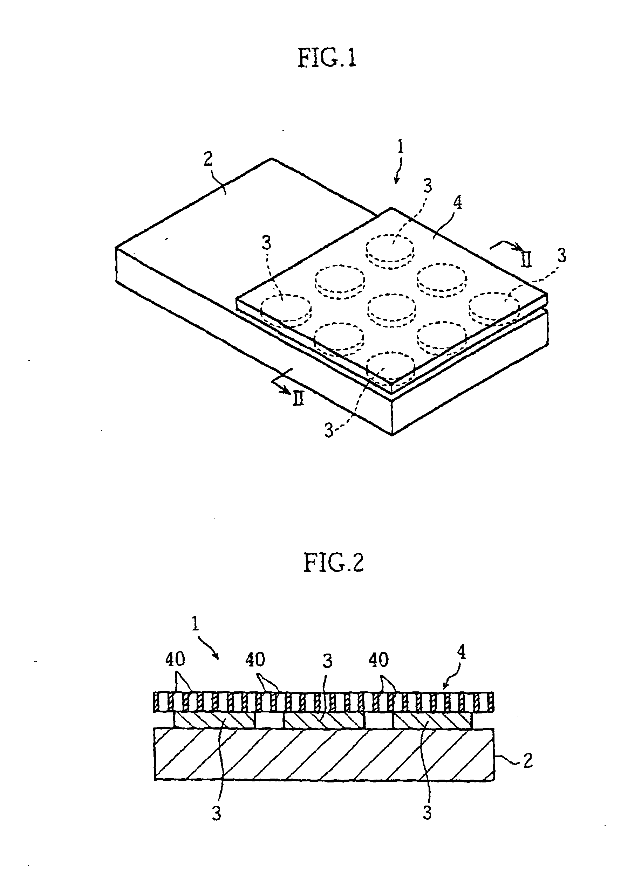

Figs. 1 and 2 illustrate an example of a test kit. A test kit 1 is designed to analyze a plurality of items using a single sample liquid, and comprises a carrier 2, a plurality of coloration pads 3, and a penetration layer 4.

The carrier 2 is made of a material with low liquid penetrability, such as PET, PC, or a like resin material.

The plurality of coloration pads 3 are arranged in a matrix. In the drawings, there are a total of nine coloration pads 3 arranged in three rows and three columns, but the number of coloration pads 3 is a design matter, and is not limited to nine.

The coloration pads 3 each react with a specific substance in a sample liquid such as urine or blood, and change color depending on the amount of the specific component. The coloration pads 3 are formed in a circular shape with a diameter of about 1 mm using a non-contact dispenser, for example. The coloration pads 3 are circular in the drawings, but may instead be formed in a rectangular shape or the like. However, the surface area of the coloration pads 3 is preferably set to be no more than 2.0 mm².

The non-contact dispenser can be one that employs an inkjet system or a dispenser discharge system. With an inkjet system, for example, reagents each containing a desired coloration agent are deposited on the carrier 2, after which each deposit of the reagents is dried to form a respective coloration pad 3. With this method, the individual coloration pad 3 can be formed to have a desired size and shape by depositing a reagent liquid a plurality of times. For example, the coloration pad can formed into a circle having a diameter of about 1mm by controlling the droplet size of the reagent liquid.

The penetration layer 4 allows liquid penetration in its thickness direction and restricts liquid penetration in its planar direction. The penetration layer is formed in a size of 2.0-15mm×2.0-15mm, for example. The penetration layer 4 may be provided by intimately attaching a penetration membrane to the coloration pads 3 by hot-press bonding, for example.

As shown well in Fig. 2, the penetration membrane is one having numerous pores 40 extending in the thickness direction. In Fig. 2, however, the size of the pores is exaggerated. What is important is that they impart thicknesswise liquid penetrability to the penetration membrane. The penetration membrane comprises a track etched membrane formed by track etching. A typical example of a track etched membrane is "Cyclopore" available from Whatman.

Track etching is a process in which a polymer film made of a polycarbonate or polyester, for example, is bombarded with neutrons, and pores are then formed by chemical etching. With this process, the pore size and porosity can be controlled by varying the neutron bombardment time or the etching treatment time. With the present invention, the penetration membrane is one having a pore size of 0.1~12µm and a porosity of 4-20%, for example.

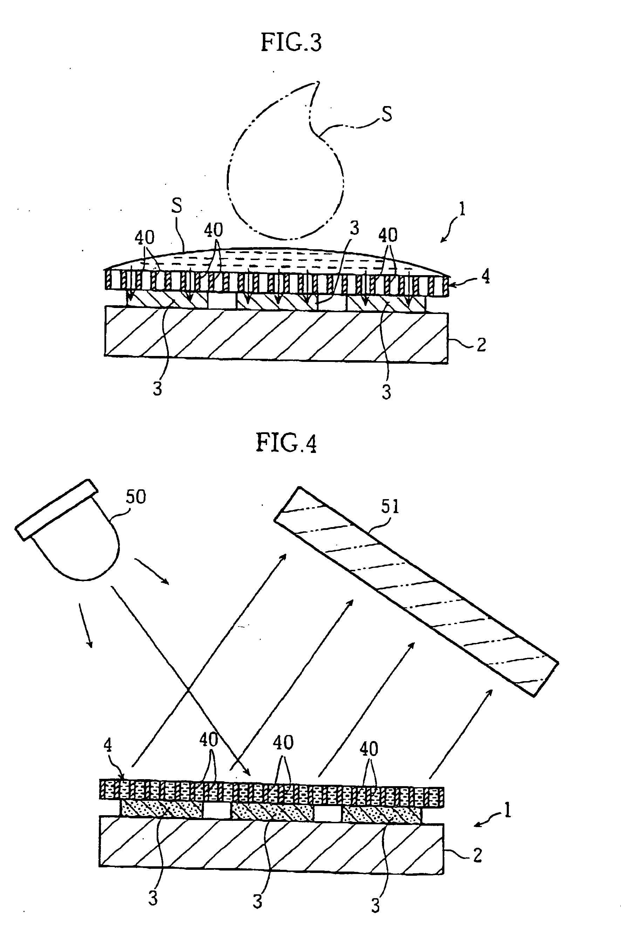

In analyzing a sample liquid with the use of the test kit 1, the penetration layer 4 of the test kit 1 is first spotted with a sample liquid S, as shown in Fig. 3. If the size of the penetration layer 4 is about 5x5mm, the amount of the sample liquid S spotted to the test kit 1 will be 4-6µL (corresponds to droplets with a diameter of about 2-3mm), for example.

With the test kit 1, since the penetration layer 4 is formed of a penetration membrane having thicknesswise liquid penetrability, the spotted sample liquid S moves downward in the thickness direction through the pores 40. As a result, the sample liquid S is guided by the pores 40 to the coloration pads 3, and the sample liquid S is supplied to the coloration pads 3.

Then, as shown in Fig. 4, the coloration agents and the specific components in the sample liquid are allowed to react for a specific length of time, after which a plurality of items are analyzed by optically observing the extent of coloration in the coloration pads 3. More specifically, a light source 50 is used to irradiate the coloration pads 3 with light, the reflected light is received by a photosensor 51, and then the extent of coloration in the coloration pads 3, or the concentration of the specific components in the sample liquid, is calculated on the basis of the amount of light received.

In this test kit, the sample liquid S penetrates thicknesswise through the penetration layer 4 due to the plurality of pores 40, and therefore does not spread out in the planar direction of the penetration layer 4. Accordingly, it is possible to suppress the formation of liquid junctions between adjacent coloration pads 3, thereby restraining the coloration component from one coloration pad 3 from mixing into an adjacent coloration pad 3; i.e., ensuring less interference between adjacent coloration pads 3. This suppression of interference is achieved merely by forming the penetration layer 4 by applying a penetration membrane having characteristic penetrability in the thickness direction. Put another way, interference can be suppressed merely by employing a different penetration membrane from that used in the past, without entailing any additional producing steps. Therefore, the test kit 1 can be produced cost-advantageously because no costly apparatus is needed to suppress interference, and no special treatment is required.

Further, if interference can be suppressed, the adjacent coloration pads 3 can be placed closer together. This affords a smaller overall size of the test kit 1, and particularly when a sample liquid is to be analyzed, this affords a smaller surface area of the region to be spotted with the sample liquid, or region to be irradiated with light (optical irradiation area). As a result, the amount of sample liquid S that is required can be reduced, so when blood is used as the sample liquid S, for example, the user has to collect less blood. Moreover, if the optical irradiation area is made smaller, the light reflected from this area can be received all at once with a C-MOS sensor, a CCD sensor, or the like, allowing the measurement device to be more compact. When the coloration pads 3 are formed by an inkjet process and in the form of a circle or rectangle having a diameter or size of about 1 mm, a plurality of coloration pads 3 for measuring a plurality of items can be put together in a small area in which only one item could be measured in the past, which means that the amount of carrier and coloration components (reagents) to be used can be reduced. This affords a reduction in material cost while also reducing the amount of waste.

In this test kit, the sample liquid was analyzed on the basis of the reflected light produced by irradiation with light from the penetration layer side, but the design may instead be such that the carrier is formed from a transparent material, and the sample liquid is analyzed on the basis of the amount of transmitted light. Further, if the carrier is transparent, the design may be such that the optical irradiation is from the back side of the carrier, and the sample liquid is analyzed on the basis of the amount of reflected or transmitted light.

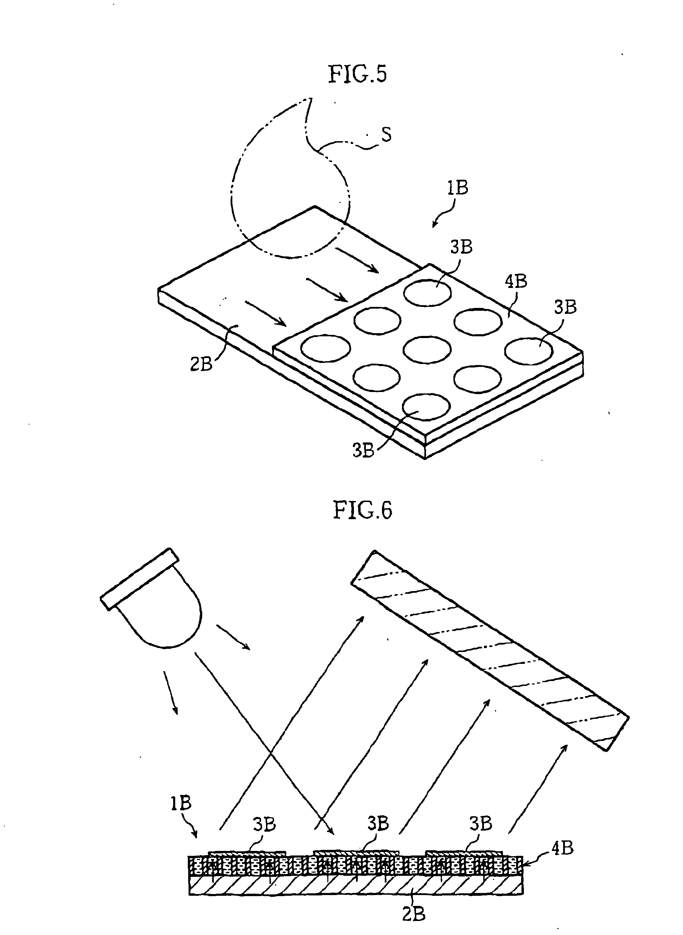

Next, a test kit according to an embodiment of the present invention will be described through reference to Figs. 5 and 6. The analytical kit 1B shown in these figures is configured such that a penetration layer 4B is formed over an absorbent carrier 2B, and a plurality of coloration pads 3B are arranged in a matrix on the penetration layer 4B.

The absorbent carrier 2B is porous, for example, and is formed so as to be liquid penetrable in at least the planar direction of the absorbent carrier 2B. Examples of absorbent carrier 2B include paper materials, foams, woven fabric materials, nonwoven fabric materials, knit materials, glass filters, and gelled materials.

The penetration layer 4B and the coloration pads 3B are formed by lamination, in that order, on the absorbent carrier 2B so as to cover approximately half of area of the absorbent carrier 2B. The penetration layer 4B is formed by bonding to the absorbent carrier 2B the same penetration membrane as that described in the test kit of Figures 1-4. The coloration pads 3B are formed over the penetration layer 4B by an inkjet process, for example.

With the test kit 1B, as shown well in Fig. 5, a sample liquid S spotted to the absorbent carrier 2B spreads out over the entire absorbent carrier 2B by capillary action. A portion of the sample liquid S held in contact with the penetration layer 4B is drawn up by the penetration layer 4B, which is combined with the absorbent carrier to provide a powerful capillary action for example, for supply to the coloration pads 3B. Thereafter, a plurality of items are optically checked in the same manner as in the test kit of Figures 1-4.

Since the test kit 1B comprises the penetration layer 4B which characteristically allows liquid to penetrate in its thickness direction, just as in the test kit of Figures 1-4, interference between adjacent coloration pads 3B can be suppressed while keeping the test kit 1B compact, all of which is achieved at low cost.

## Claims

1. A test kit comprising: a water absorbent carrier (2B) for receiving, a sample liquid (S) and for spreading it out, a penetration layer (4B) for drawing up the sample liquid, and a plurality of coloration pads (3B) held in contact with the penetration layer,
wherein the penetration layer (4B) and the plurality of coloration pads (3B) are laminated in this order on the water absorbent carrier (2B);
wherein the penetration layer (4B) is arranged for feeding the sample liquid (5) to each of the coloration pads; and
wherein the penetration layer (4B) is formed with a plurality of thicknesswise extending pores (40) for allowing the sample liquid (S) to penetrate thicknesswise of said penetration layer while restricting the sample liquid from spreading in a planar direction of the penetration layer;
**characterized in that** the plurality of pores (40) have a size of 0.1-12 µm, **in that** the penetration layer (4B) is a track etched membrane, **in that** the plurality of coloration pads (3B) are formed within a specific region, the surface area of the specific region being 2.0~15mm × 2.0~15mm, and **in that** the surface area of the specific region accounted for by each respective coloration pad (3B) is no more than 2.0 mm².

2. The test kit according to Claim 1, wherein the penetration layer (4B) has a porosity of 4-20 vol%.

3. The test kit according to Claim 1, wherein the plurality of coloration pads (3B) are arranged in a matrix.

4. The test kit according to Claim 1, wherein at least two of the plurality of coloration pads (3B) differ from each other with respect to coloration components for allowing measurement of a plurality of items.

## Patentansprüche

1. Testanordnung bzw. -Kit, mit: einem wasserabsorbierenden Träger (2B), um eine Probenflüssigkeit (S) aufzunehmen und zu verbreiten, einer Eindringschicht (4B), um die Probenflüssigkeit aufzusaugen, und mehreren Färbekissen (3B), die mit der Eindringschicht in Kontakt gehalten werden,
wobei die Eindringschicht (4B) und die mehreren Färbekissen (3B) in dieser Reihenfolge auf den wasserabsorbierenden Träger (2B) laminiert sind;
wobei die Eindringschicht (4B) dazu ausgelegt ist, die Probenflüssigkeit (5) jedem der Färbekissen zuzuführen; und
wobei die Eindringschicht (4B) mit einer Anzahl von in Dickenrichtung verlaufenden Poren (40) ausgebildet ist, um der Probenflüssigkeit (S) zu ermöglichen, in Dickenrichtung der Eindringschicht einzudringen, während eine Verbreitung der Probenflüssigkeit in einer ebenen bzw. planaren Richtung der Eindringschicht eingeschränkt ist;
**dadurch gekennzeichnet, dass** die Anzahl von Poren (40) eine Größe im Bereich 0,1 bis 12 µm aufweist bzw. aufweisen, dass die Eindringschicht (4B) eine bahngeätzte Membran ist, dass die mehreren Färbekissen (3B) in einem bestimmten Bereich ausgebildet sind, wobei der Oberflächeninhalt des bestimmten Bereichs im Bereich von 2,0 bis 15 mm × 2,0 bis 15 mm liegt, und dass der Oberflächeninhalt des bestimmten Bereichs, der von jedem entsprechenden Färbekissen (3B) eingenommen wird, nicht größer als 2,0 mm² ist.

2. Testanordnung nach Anspruch 1, wobei die Eindringschicht (4B) eine Porosität im Bereich von 4 bis 20 Vol.-% besitzt.

3. Testanordnung nach Anspruch 1, wobei die mehreren Färbekissen (3B) in einer Matrix angeordnet sind.

4. Testanordnung nach Anspruch 1, wobei sich wenigstens zwei der mehreren Färbekissen (3B) in Bezug auf die Färbekomponenten voneinander unterscheiden, um eine Messung mehrerer Elemente zu ermöglichen.

## Revendications

1. Kit de test comprenant : un support absorbant l'eau (2B) pour recevoir un échantillon liquide (S) et pour le disperser, une couche de pénétration (4B) pour attirer l'échantillon liquide vers le haut, et une pluralité de pastilles de coloration (3B) maintenues en contact avec la couche de pénétration,
dans lequel la couche de pénétration (4B) et la pluralité de pastilles de coloration (3B) sont lamifiées dans cet ordre sur le support absorbant l'eau (2B) ;
dans lequel la couche de pénétration (4B) est agencée pour alimenter en échantillon liquide (5) chacune des pastilles de coloration ; et
dans lequel la couche de pénétration (4B) comporte une pluralité de ports (40) s'étendant dans le sens de l'épaisseur pour permettre à l'échantillon liquide (S) de pénétrer dans le sens de l'épaisseur de ladite couche de pénétration tout en empêchant l'échantillon liquide de se disperser dans une direction plane de la couche de pénétration ;
**caractérisé en ce que** la pluralité de ports (40) ont une taille de 0,1 ~ 12 µm, **en ce que** la couche de pénétration (4B) consiste en une membrane dans laquelle des traces sont gravées, **en ce que** la pluralité de pastilles de coloration (3B) sont formées dans une région spécifique, l'aire de surface de la région spécifique étant de 2,0 ~ 15 mm x 2,0 ~ 15 mm, et **en ce que** l'aire de surface de la région spécifique représentée par chaque pastille de coloration (3B) respective est inférieure ou égale à 2,0 mm²_{.}

2. Kit de test selon la revendication 1, dans lequel la couche de pénétration (4B) a une porosité de 4 ~ 20 % en volume.

3. Kit de test selon la revendication 1, dans lequel la pluralité de pastilles de coloration (3B) sont agencées en une matrice.

4. Kit de test selon la revendication 1, dans lequel au moins deux de la pluralité de pastilles de coloration (3B) diffèrent l'une de l'autre en ce qui concerne les composants de coloration pour permettre la mesure d'une pluralité d'articles.
